# EUROPÄISCHE PATENTSCHRIFT

(11) **EP 1 796 702 B1**
(45) Veröffentlichungstag und Bekanntmachung des Hinweises auf die Patenterteilung: **11.11.2009**
(21) Anmeldenummer: 05791751.0
(22) Anmeldetag: 05.10.2005
(51) Int. Cl.: A61K 36/53, A61K 133/00

(54) **VERWENDUNG VON LAVENDELÖL ZUR PROPHYLAXE UND BEHANDLUNG VON SOMATISIERUNGSSTÖRUNGEN UND VON POSTTRAUMATISCHER STRESSERKRANKUNG**
USE OF LAVENDER OIL FOR THE PROPHYLAXIS AND TREATMENT OF SOMATIZATION DISORDERS AND POSTTRAUMATIC STRESS DISORDER
UTILISATION D'HUILE DE LAVANDE POUR LA PROPHYLAXIE ET LE TRAITEMENT DE TROUBLES SOMATIQUES ET D'ETAT DE STRESS POST-TRAUMATIQUE

(30) Priorität: 06.10.2004 DE 102004048716
(43) Veröffentlichungstag der Anmeldung: 20.06.2007
(73) Patentinhaber: Dr. Willmar Schwabe GmbH & Co. KG, 76227 Karlsruhe (DE)
(72) Erfinder: DIENEL, Angelika, 76229 Karlsruhe (DE); NÖLDNER, Michael, 76139 Karlsruhe (DE)
(74) Vertreter: Adam, Holger
(86) Internationale Anmeldenummer: PCT/EP2005/010732
(87) Internationale Veröffentlichungsnummer: WO 2006/037629

(56) Entgegenhaltungen:
- EP-A- 0 891 777
- GB-A- 2 355 189
- US-A1- 2002 136 788
- ALAKBAROV, F.: "Aromatic Herbal Baths of the Ancients" THE JOURNAL OF THE AMERICAN BOTANICAL COUNCIL, Bd. 57, 2003, Seiten 40-49, XP002359006
- ANONYMOUS: "St. Gallener Ganzheitsmedizin-Fibel" INTERNET ARTICLE, [Online] 25. Juli 2004 (2004-07-25), Seiten 1-14, XP002359007 Gefunden im Internet: URL:HTTP://WWW.GANZMED.INFO/MEDIZINDB/DEFA ULT.ASP?MITTEL=OELE&START=A&ENDE=M> [gefunden am 2005-12-13]

## Beschreibung

Die vorliegende Erfindung betrifft die prophylaktische und therapeutische Verwendung von Lavendelöl zur Behandlung von Somatisierungsstörungen und von PTSD (posttraumatischer Stresserkrankung) sowie Lavendelöl enthaltende Arzneimittel und diätetische Lebensmittel, vorzugsweise in Form einer Kapsel als orale Darreichungsform.

Als Neurasthenie bezeichnet man eine erworben Nervosität mit den Symptomen rasche Ermüdbarkeit, körperliche Schwäche, Kopf- und Gliederschmerzen, vegetative Übererregbarkeit, Affektlabilität, Merk- und Konzentrationsschwäche, Reizbarkeit, Stimmungsschwankungen und Schlafstörungen (Roche Lexikon, Medizin, 4. Auflage 1998). Alakbarov, F. ("Aromatic Herbal Baths of the Ancients" THE JOURNAL OF THE AMERICAN BOTANICAL COUNCIL, Bd. 57, 2003, Seiten 40-49) offenbart die äußerliche Verwendung von Lavendelöl zur Behandlung von Neurasthenie.

Als Somatisierungsstörung bezeichnet man das Vorliegen von multiplen, wiederholt auftretenden und häufig wechselnden körperlichen Symptomen. Die Symptome können sich auf jedes Körperteil oder jedes System des Körpers beziehen.

Neurasthenie und Somatisierungsstörungen können sich aufgrund verschiedener Ursachen ausbilden, wobei Stress häufig ein auslösender Faktor ist.

Unter Stressreaktion versteht man heute üblicherweise die körperlichen Veränderungen, die im Zusammenhang mit externen unerwünschten Stimuli auftreten und eine Vielzahl von physiologischen Reaktionen beinhalten, die zum Ziel haben die zum Überleben wichtigen Funktionen aufrechtzuerhalten und zu stimulieren (G. A. Carrasco et al. (2003), Eur. J. Pharmacol. 463, 235 - 272). Allerdings wirken die durch Stress aktivierten physiologischen Systeme nicht nur schützend und funktionserhaltend, sondern können auch schädliche Folgen haben (B. S. McEwen (1998), New Engl. J. Med. 338, 171 - 179). So ist bekannt, dass Stress eine Vielzahl von Krankheiten negativ beeinflusst und chronische Einwirkung von Stress auch Krankheiten auslösen kann (S. Sephton et al. (2003), Brain Behav. Immun. 17, 321 - 328; P. H. Black et al. (2002), J. Psychosom. Res. 52, 1 - 23). Zu diesen stress-assoziierten Erkrankungen gehören außer den genannten Erkrankungen z. B. auch Erkrankungen des Herz-Kreislauf Systemes, des Muskel- und Skelettsystemes oder Störungen des Immunsystemes sowie die posttraumatische Stresserkrankung (PTSD).

Die derzeit zur Verfügung stehenden Arzneimittel zur Behandlung von Somatisierungsstörungen und PTSD umfassen Anxiolytika wie z. B. verschiedene Benzodiazepine, Neuroleptika sowie verschiedene Antidepressiva. Die Wirksamkeit dieser Mittel ist jedoch begrenzt. Desweiteren sind sie auch mit erheblichen Nebenwirkungen verbunden. So führt z. B. die chronische Verwendung von Benzodiazepinen zu Abhängigkeit, während die Neuroleptika sehr unangenehme sogenannte Früh- oder Spätdyskinesien auslösen können. Der Gebrauch von Antidepressiva führt abhängig vom verwendeten Arzneimittel häufig zu vegetativen Störungen wie z. B. Mundtrockenheit, Tremor, Müdigkeit, oder zu sexuellen Funktionsstörungen bis zur Anorgasmie.

Aufgabe der vorliegenden Erfindung ist es somit ein Mittel bereitzustellen, das wirksam zur Prophylaxe und/oder Behandlung von Somatisierungsstörungen und PTSD verwendet werden kann und weitgehend frei von Nebenwirkungen ist.

Diese Aufgabe wird durch die orale Verwendung von Lavendelöl gelöst.

Echter Lavendel (Lavendula angustifolia MILL.) wächst als Halbstrauch bis etwa 60 cm hoch. Das Verbreitungsgebiet reicht von den Kanaren durch das ganze Mittelmeergebiet bis Vorderindien. Als Inhaltsstoffe sind v. a. ätherische Öle in den oberirdischen Teilen (hauptsächlich Monoterpene), sowie Kaffeesäure und deren Depside in den Blättern beschrieben. Die aus Lavendel hergestellten ätherischen Öle werden traditionell für kosmetische Produkte, aber auch zu therapeutischen Zwecken z. B. in der Aromatherapie verwendet. So sind für Lavendelöl antibakterielle, antifungale, spasmolytische, sedative und antidepressive Wirkungen beschrieben (H. M. A. Cavanagh et al. (2002), Phytother. Res. 16, 301 - 308).

In Deutschland sind Zubereitungen aus Lavendelblüten in Form von Teeaufgüssen, als Extrakt sowie als Badezusatz für die Indikationen Unruhezustände, Einschlafstörungen, funktionelle Oberbauchbeschwerden, Meteorismus und in der Balneotherapie positiv monographiert (Monographie der Kommission E des ehemaligen deutschen Bundesgesundheitsamts).

Es wurde nun überraschend festgestellt, dass die orale Applikation von Lavendelöl bei Patienten mit Somatisierungsstörungen und/oder posttraumatischer Stresserkrankung zu einer deutlichen Verbesserung verschiedener krankheitsbezogener Symptome führt. Zusätzlich wurden im Tierversuch stressinduzierte Verhaltensänderungen durch orale Gabe von Lavendelöl signifikant gehemmt. Gemäß der vorliegenden Erfindung kann die orale Einnahme von Lavendelöl somit für die Therapie von Somatisierungsstörungen und PTSD verwendet werden. Derartige Wirkungen wurden bisher für Lavendelöl nicht beschrieben und waren aufgrund der bisher für Lavendelöl bekannten pharmakologischen und klinischen Effekte nicht zu erwarten.

Die Wirksamkeit von Lavendelöl wurde an Patienten getestet, die an Neurasthenie und/oder posttraumatischer Stresserkrankung (PTSD) und/oder einer Somatisierungsstörung litten. Nach 6 Wochen Therapie mit tägl. 80 mg Lavendelöl verbesserten sich die Kernsymptome der Neurasthenie, der PTSD und der Somatisierungsstörung. Die Lebensqualität der Patienten zeigte einen deutlichen Zuwachs (vgl. Beispiel 1).

Die stress-mindernden Effekte von Lavendelöl wurden an Ratten im sogenannten "Forced Swimm Stress" Modell nachgewiesen (R. D. Porsolt et al. (1978), Eur. J. Pharmacol. 47, 379 - 391). Die in dieser Versuchsanordnung experimentell an Ratten ausgelöste Stressreaktion ist ein anerkanntes Verfahren, um stressinduziertes Verhalten zu testen. Das Prinzip des verwendeten Testsystemes beruht darauf, dass Ratten, die in einen wassergefüllten Glaszylinder gesetzt werden, den sie nicht eigenständig verlassen können, nach einer kurzen Schwimmzeit in eine Ruheposition (Immobilisationszeit) fallen. Diese Immobilität wird als Reaktion auf das Erkennen der Ausweglosigkeit der Situation gedeutet (vgl. Beispiel 2).

Lavendelöl kann nach bekannten Herstellungsverfahren, vorzugsweise durch Wasserdampfdestillation von frisch geernteten Lavendelblüten hergestellt werden.

Das Lavendelöl kann abgefüllt in Kapseln aus Gelatine, Cellulosederivaten, oder anderen zur Verkapselung geeigneten Materialien oder auch als Lösung, vorzugsweise oral verabreicht werden.

Zur Herstellung von Kapseln wird das Öl mit geeigneten pharmazeutisch akzeptablen Hilfstoffen wie z. B. mittelkettigen Triglyzeriden, pflanzlichen Ölen (z. B. Sonnenblumenöl, Sojabohnenöl, Weizenkeimöl und andere) gemischt und in Kapseln abgefüllt. Gegegebenenfalls werden der Mischung weitere Hilfsstoffe z. B. Stabilisatoren zugefügt.

Die Dosierung erfolgt dabei so, dass pro Tag 10 mg bis 2 g, bevorzugt 20 bis 500 mg und besonders bevorzugt 50 bis 100 mg Lavendelöl zugeführt werden.

### Beispiele

### Beispiel 1: Wirksamkeit von Lavendelöl an Menschen

50 Patienten, die an Neurasthenie und/oder posttraumatischer Stresserkrankung (PTSD) und/oder einer Somatisierungsstörung litten, wurden sechs Wochen mit täglich 80 mg Lavendelöl gemäß Europäischem Arzneibuch, Ausgabe 4 (WS^{®} 1265) behandelt. Die Verbesserung der Symptomatik wurde mit folgenden anerkannten Testverfahren gemessen und dokumentiert: Symptom Checkliste (SCL 90), State-Trait-Anxiety Inventory (STAI; A. M. Sesti (2000), QoL Newsletter 25, 15 - 16), Depressions-Skala (D-S), Maslach Burnout Inventory (MBI), 36 Item Short Form Survey (SF-36), State check und Schlaftagebuch. Dazu kamen die Patienten in die Klinik und wurden von einem Arzt nach den Symptomen befragt (Fremdbeurteilung) bzw. die Patienten füllten die vorgegebenen Fragebögen aus (Selbstbeurteilung). Änderungen im Vergleich vor und nach sechswöchiger Therapie wurden mit dem Wilcoxon Signed Rank Rest überprüft und waren für den überwiegenden Teil der Symptome statistisch signifikant gebessert.

Nach 6 Wochen Therapie mit täglich 80 mg Lavendelöl WS^{®} 1265 verbesserte sich die Unruhe bei 29 (61,7 %) der Patienten und die Angst bei 21 (44,7 %) der Patienten (state check, Irrtumswahrscheinlichkeit p < 0,001 für beide Symptome, hier und im folgenden Wilcoxon Signed Rank Test). Sowohl die State Anxiety (Verbesserung von 4,5 ± 10,7 Punkten p = 0,005) als auch die Trait Anxiety (Verbesserung von 7,4 ± 8,9 Punkten, p < 0,001) wurden vermindert. Eine Verbesserung der Schlafstörungen zeigte sich bei 24 (51,5 %) der Patienten (state check, Irrtumswahrscheinlichkeit p < 0,001) und vier wichtige Items im Schlaftagebuch zeigten eine deutliche Besserung während der Behandlungsphase. Die Aufwachhäufigkeit verbesserte sich in Woche 1 von 1,9 ± 0,7 um 0,2 ± 0,6 Mal (p = 0,004), die Aufwachdauer verminderte sich von 35,6 ± 28,0 um 12,8 ± 24.3 Minuten (p < 0,001), die Gesamtschlafdauer wurde während der Therapie in Woche 1 von 379,6 ± 59,1 um 16,6 ± 43,0 Minuten verlängert (p = 0,024), die Schlafeffizienz verbesserte sich in Woche 1 von 77,4 ± 10,2 % um 3,9 ± 8,1 % (p = 0,001). Außerdem zeigte sich eine leichte Besserung der morgendlichen Stimmung sowie der Morgenmüdigkeit und eine leichte Zunahme der Leistungsfähigkeit (morgendliche Stimmung: 3,0 ± 0,5 in Woche 1, Verbesserung von 0,2 ± 0,6 Punkten, Morgenmüdigkeit: 3,4 ± 0,7 in Woche 1, Verbesserung von 0,3 ± 0,8 Punkten, Effizienz: 3,0 ± 0,7 in Woche 1, Verbesserung von 0,2 ± 0,6 Punkten). Die depressive Stimmung von 27 (57,4 %) Patienten verbesserte sich nach 6-wöchiger Therapie mit Lavendelöl WS^{®} 1265 (state check, p < 0,001) und die Punktzahl der Depressions-Skala (D-S) verminderte sich um 5,5 ± 7,0 Punkte (p < 0,001). Alle Subscores des SCL-90-R und, als Folge, der Global Severity Index, Positive Symptom Total und der Positive Symptom Distress Index reduzierten sich bis Woche 6 (Verbesserung von 0,4 ± 0,3 (GSI), 14 ± 12,4 (PST) und 0,4 ± 0,4 (PSDI) Punkte, p < 0,001 für alle drei global scores. Der Physical Health Score und der Mental Health Score des SF-36 erhöhten sich deutlich nach 6-wöchiger Behandlung um 9,8 ± 15,7 bzw. um 20,8 ± 22,3 Punkte (p < 0,001 für beide Scores). Die Studienergebnisse zeigen demzufolge eine deutliche Verbesserung der Symptomatik bei Neurasthenie, Somatisierungsstörungen und anderen stressbedingten Erkrankungen durch die Einnahme von Lavendelöl.

### Beispiel 2: Wirksamkeit von Lavendelöl an Ratten

Um die stress-mindernden Effekte von Lavendelöl zu testen wurden Ratten über einen Zeitraum von insgesamt 9 Tagen einmal täglich mit verschieden hohen Dosierungen Lavendelöl gemäß Europäischem Arzneibuch, Ausgabe 4 (WS^{®} 1265) behandelt. Zum Vergleich wurden einige Tiere entweder mit dem in der Stress-Therapie verwendeten Trizyklischem Antidepressivum Imipramin oder nur mit einer wirkungslosen Kontrolllösung behandelt. Am 7. Behandlungstag wurden die Tiere zur Eingewöhnung für 15 Minuten in den wassergefüllten Glaszylinder gesetzt. Am letzten Behandlungstag wurden die Tiere erneut für einen Zeitraum von 5 Minuten in den Meßzylinder gesetzt und als Maß der Stressreaktion die Zeit der Immobilität gemessen. Orale Applikation von Lavendelöl über neun Tage in einer Dosierung von 10 bis 100 mg/kg führt zu einer deutlichen, signifikanten Reduktion der Immobilisationszeit.

| **Substanz** | **Dosis** mg/kg peroral | **Immobilisationszeit** Sekunden | **Hemmung** % |
|---|---|---|---|
| Kontrolle | | 146 ± 11 | 0 |
| Lavendelöl | 1 | 149 ± 8 | 0 |
| Lavendelöl | 3 | 136 ± 12 | 7 |
| Lavendelöl | 10 | 116 ± 13 * | 21 |
| Lavendelöl | 30 | 83 ± 5 * | 43 |
| Lavendelöl | 100 | 80 ± 17 * | 45 |
| Imipramin | 30 | 48 ± 6 * | 67 |

| | | | |
|---|---|---|---|
| * Irrtumswahrscheinlichkeit p < 0,05 versus Kontrolle | | | |

### Beispiel 3: Kapseln

Die zur Herstellung von ca. 10.000 Kapseln erforderliche Menge von 800 g Lavendelöl wird in einem dicht verschlossenen, inerten Behältnis (z. B. Rührwerksbehälter aus Edelstahl) mit 1200 g mittelkettiger Triglyceride unter Rühren 15 min gemischt. Je 200 mg der homogenen flüssigen Mischung werden mit einer geeigneten Kapselfüllmaschine in Gelatinekapseln abgefüllt. Im Fall von Hartkapseln (z. B. aus Gelatine oder Cellulosederivaten) werden die Kapseln nach der Befüllung durch eine Banderole verschlossen. Im Fall von Kapseln aus Weichgelatine werden die Kapseln in einem Arbeitsgang befüllt und verschlossen.

## Patentansprüche

1. Arzneimittel oder diätetisches Lebensmittel als orale Darreichungsform zur Anwendung in der Prophylaxe oder Behandlung von Somatisierungsstörungen und von PTSD (posttraumatische Stresserkrankung), **gekennzeichnet durch** einen Gehalt an Lavendelöl und ggf. pharmazeutisch akzeptablen Hilfsstoffen.

2. Arzneimittel oder diätetisches Lebensmittel als orale Darreichungsform nach Anspruch 1, in Form einer Kapsel.

3. Arzneimittel oder diätetisches Lebensmittel als orale Darreichungsform in Form einer Kapsel nach Anspruch 2, wobei die Kapsel aus einer Füllmasse aus Lavendelöl und pharmazeutisch akzeptablen Hilfsstoffen sowie einer geeigneten Hülle aus Gelatine oder Cellulosederivaten besteht.

4. Verwendung von Lavendelöl zur Herstellung eines Arzneimittels oder diätetischen Lebensmittels zur Prophylaxe oder Behandlung von Somatisierungsstörungen und von PTSD (posttraumatische Stresserkrankung), wobei das Lavendelöl oral verabreicht wird.

5. Verwendung nach Anspruch 4, wobei das Lavendelöl in Form einer Kapsel als orale Darreichungsform verabreicht wird.

6. Verwendung nach Anspruch 5, wobei die Kapsel aus einer Füllmasse aus Lavendelöl und pharmazeutisch akzeptablen Hilfsstoffen sowie einer geeigneten Hülle aus Gelatine oder Cellulosederivaten besteht.

## Claims

1. Medicament or dietetic food product as an oral administration form for the application in the prophylaxis or treatment of somatization disorders and PTSD (posttraumatic stress disease), **characterized by** a content of lavender oil and optionally pharmaceutically acceptable adjuvants.

2. Medicament or dietetic food product as an oral administration form according to claim 1 in the form of a capsule.

3. Medicament or dietetic food product as an oral administration form in the form of a capsule according to claim 2 with the capsule consisting of a filling material of lavender oil and pharmaceutically acceptable adjuvants as well as a suitable shell made of gelatine or cellulose derivatives.

4. Use of lavender oil for the preparation of a medicament or dietetic food product for the prophylaxis or treatment of somatization disorders and PTSD (posttraumatic stress disease), wherein the lavender oil is administered orally.

5. Use according to claim 4, wherein the lavender oil is administered in the form of a capsule as an oral administration form.

6. Use according to claim 5, wherein the capsule consists of a filling material of lavender oil and pharmaceutically acceptable adjuvants as well as a suitable shell made of gelatine or cellulose derivatives.

## Revendications

1. Médicament ou aliment diététique en préparation orale destiné à une utilisation pour la prophylaxie ou le traitement de troubles somatiques et d'état de stress post-traumatique (ESPT), **caractérisé en ce qu'**il a une teneur en huile de lavande et éventuellement en excipients pharmaceutiquement acceptables.

2. Médicament ou aliment diététique en préparation orale selon la revendication 1 sous forme de capsule.

3. Médicament ou aliment diététique en préparation orale sous forme de capsule selon la revendication 2, le capsule consistant en une matière de charge de lavande et des excipients pharmaceutiquement acceptables et une enveloppe appropriée constituée de gélatine ou de dérivés de cellulose.

4. Utilisation d'huile de lavande pour la fabrication d'un médicament ou d'un aliment diététique destiné à la prophylaxie ou le traitement des troubles somatiques et d'état de stress post-traumatique (ESPT), l'huile de lavande étant administrée par voie orale.

5. Utilisation selon la revendication 4, dans laquelle l'huile de la lavande sous forme de capsules est administrée en préparation orale.

6. Utilisation selon la revendication 5, dans laquelle la capsule consiste en une matière de charge de lavande et des excipients pharmaceutiquement acceptables et une enveloppe appropriée constituée de la gélatine ou de dérives de cellulose.
